# EUROPEAN PATENT APPLICATION

(11) **EP 4 825 407 A1**
(43) Date of publication of application: **21.10.2026**
(21) Application number: 26171571.8
(22) Date of filing: 10.04.2026
(51) Int. Cl.: A61M 1/38, A61M 1/34

(54) **DETERMINATION OF PLATELET CONCENTRATION OF BLOOD AND ELIGIBILITY FOR PLATELET COLLECTION PROCEDURE**

(30) Priority: 14.04.2025 US 202563788316 P
(71) Applicant: Fenwal, Inc., Lake Zurich, IL 60047 (US)
(72) Inventor: KUSTERS, Benjamin E., Lake Zurich, 60047 (US); MIN, Kyungyoon, Lake Zurich, 60047 (US)
(74) Representative: Maikowski & Ninnemann Patentanwälte Partnerschaft mbB

(57) **Abstract**

A blood processing device includes a pump system, a valve system, and a controller programmed to control the operation of the pump system and the valve system to execute a platelet collection procedure for a blood source. The controller is programmed to determine the concentration of platelets circulating in the blood of the blood source during the platelet collection procedure (without using a platelet pre-count) and compare the concentration of platelets circulating in the blood of the blood source during the platelet collection procedure to a minimum threshold. The controller allows the platelet collection procedure to continue when the concentration of platelets circulating in the blood of the blood source exceeds the minimum threshold, while either generating an alert or automatically terminating the platelet collection procedure when the concentration of platelets circulating in the blood of the blood source does not exceed the minimum threshold.

## Description

### Background

### Field of the Disclosure

The present disclosure relates to determination of the platelet concentration of a fluid. More particularly, the present disclosure relates to determination of the platelet concentration of the blood of a blood source and whether the blood source is eligible for a platelet collection procedure.

### Description of Related Art

Various blood processing systems now make it possible to collect particular blood constituents, instead of whole blood, from a blood source. Typically, in such systems, whole blood is drawn from a blood source, the particular blood component or constituent is separated, removed, and collected, and the remaining blood constituents are returned to the blood source. Removing only particular constituents is advantageous when the blood source is a living subject (e.g., a human or animal), because potentially less time is needed for their body to return to pre-procedure levels, thus allowing for more iterations of a procedure (e.g., a platelet donation procedure) to be repeated in a given amount of time for the source compared to when whole blood is collected. This increases the overall supply of blood constituents, such as plasma and platelets, made available for transfer and/or therapeutic treatment.

Estimating a blood source's circulating platelet concentration is an important part of any platelet collection procedure. The source's platelet concentration is needed for the blood processing system to calculate the number of platelets collected (yield) and for system safeguards to ensure that the source's platelet count does not drop below acceptable limits, such as 150e3 platelets/µL. Currently, blood processing systems employ complex mathematical algorithms to estimate collection yields and source concentrations based on a platelet pre-count of the source that is calculated using a laboratory cell counter or the like. Sometimes, a sample of the blood of a source is taken shortly before a platelet collection procedure is begun, with the platelet pre-count not being available until later in the platelet collection procedure. Upon determining that the platelet concentration is below a particular threshold, an operator of the system will manually terminate the procedure.

Although effective, this conventional approach has a number of disadvantages and could be improved by an approach that more quickly determines the concentration of platelets circulating in the blood of a source, eliminates operator involvement in termination of a procedure for an ineligible source, and allows for execution of a platelet collection procedure when a platelet pre-count is unavailable or difficult to obtain (e.g., in a mobile setting).

### Summary

There are several aspects of the present subject matter which may be embodied separately or together in the devices and methods described and claimed below. These aspects may be employed alone or in combination with other aspects of the subject matter described herein, and the description of these aspects together is not intended to preclude the use of these aspects separately or the claiming of such aspects separately as set forth in the claims appended hereto.

In one aspect, a blood processing device includes a pump system, a valve system, and a controller programmed to control the operation of the pump system and the valve system to execute a platelet collection procedure for a blood source. The controller is programmed to determine a concentration of platelets circulating in blood of the blood source during the platelet collection procedure without using a platelet pre-count and compare the concentration of platelets circulating in the blood of the blood source during the platelet collection procedure to a minimum threshold. The controller allows the platelet collection procedure to continue when the concentration of platelets circulating in the blood of the blood source exceeds the minimum threshold. The controller generates an alert or automatically terminate the platelet collection procedure when the concentration of platelets circulating in the blood of the blood source does not exceed the minimum threshold.

In another aspect, a controller-implemented method is provided for executing a platelet collection procedure for a blood source. The method includes the controller determining a concentration of platelets circulating in the blood of the blood source during the platelet collection procedure without using a platelet pre-count and comparing the concentration of platelets circulating in the blood of the blood source during the platelet collection procedure to a minimum threshold. The controller allows the platelet collection procedure to continue when the concentration of platelets circulating in the blood of the blood source exceeds the minimum threshold. The controller instead generates an alert or automatically terminates the platelet collection procedure when the concentration of platelets circulating in the blood of the blood source does not exceed the minimum threshold.

### Brief Description of the Drawings

Fig. 1 is a perspective view of an exemplary hardware component or blood processing device of a blood processing system according to an aspect of the present disclosure;
Fig. 2 is a schematic view of an exemplary disposable component or fluid flow circuit mounted to the hardware component of Fig. 1 to complete a blood processing system according to an aspect of the present disclosure;
Fig. 3 illustrates the blood processing system of Fig. 2 implementing a draw phase of an exemplary platelet collection procedure;
Fig. 4 is a flowchart illustrating an exemplary approach to calculation of the concentration of platelets in the blood of a source by a controller of the hardware component of Fig. 1, according to an aspect of the present disclosure; and
Fig. 5 is a flowchart illustrating an exemplary approach to determination of the eligibility of a blood source for a platelet collection procedure by the controller of the hardware component of Fig. 1, according to an aspect of the present disclosure.

### Description of the Illustrated Embodiments

The embodiments disclosed herein are for the purpose of providing an exemplary description of the present subject matter. They are, however, only exemplary, and the present subject matter may be embodied in various forms. Therefore, specific details disclosed herein are not to be interpreted as limiting the subject matter as defined in the accompanying claims.

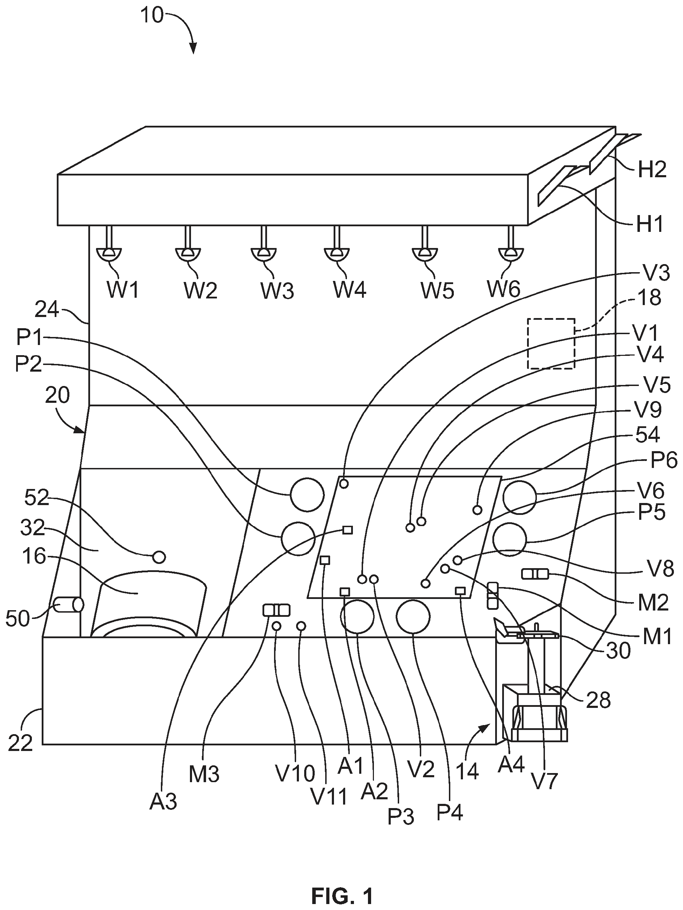

Figs. 1-3 show components of a blood processing system that embodies various aspects of the present subject matter. Use of the system for separating blood into two or more components and collecting at least one of the components (namely, platelets) will be described herein, though it should be understood that systems according to the present disclosure can be used for processing a variety of different biological fluids.

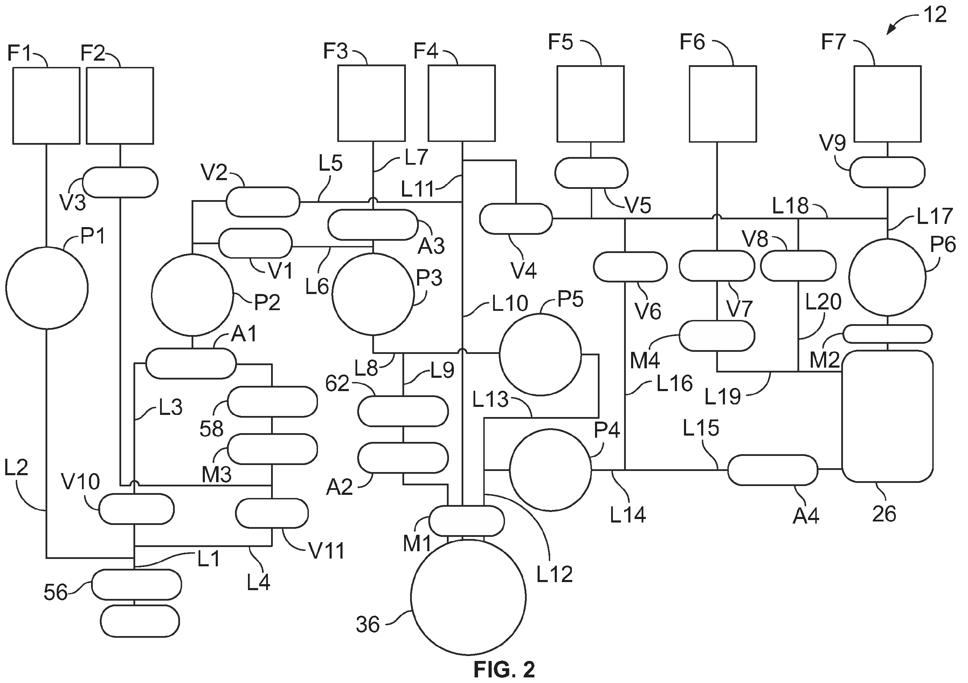

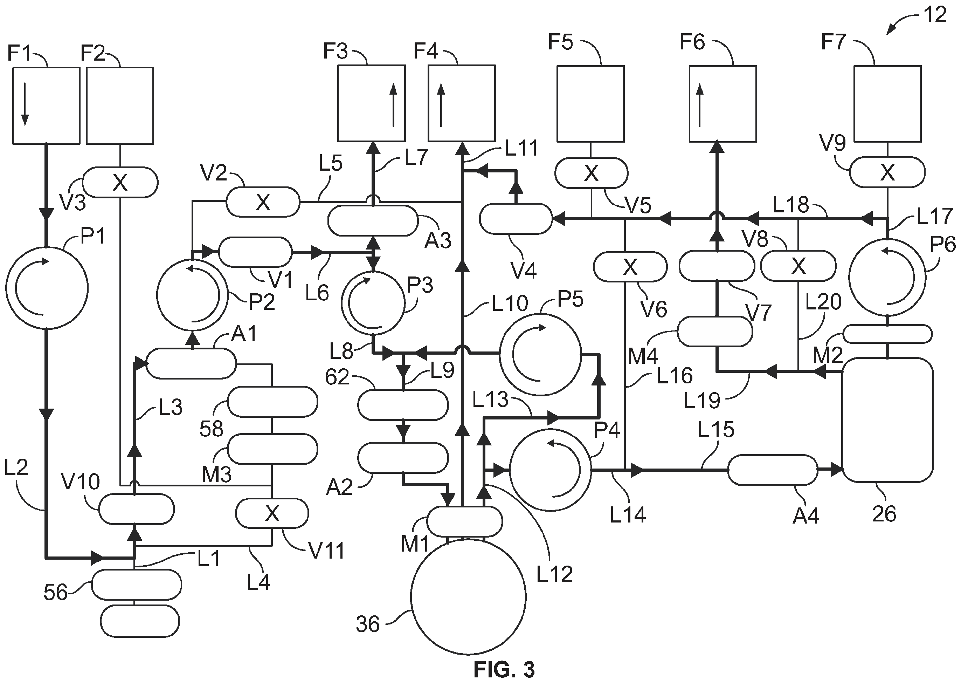

Generally speaking, the system includes two principal components, a durable and reusable blood processing device 10 (Fig. 1) and a disposable fluid flow circuit 12 (Figs. 2 and 3). The illustrated blood processing system is configured generally in accordance with the system described in PCT Patent Application Publication No. WO 2018/053217 A1, which is hereby incorporated herein by reference. The blood processing device 10 of Fig. 1 includes a spinning membrane separator drive unit 14, a centrifuge or centrifugal separator 16, additional components that control fluid flow through the disposable fluid flow circuit 12, and a controller 18, which governs the operation of the other components of the blood processing device 10 to perform a blood processing procedure. While the principles described herein may be employed when using the blood processing device 10 of Fig. 1, it should be understood that these same principles may be applied to other blood processing devices, including devices employing single separation technologies or approaches.

### I. The Durable Blood Processing Device

The blood processing device 10 (Fig. 1) is configured as a durable item that is capable of long-term use. It should be understood that the blood processing device 10 of Fig. 1 is merely exemplary of one possible configuration and that blood processing devices according to the present disclosure may be differently configured.

In the illustrated embodiment, the blood processing device 10 is embodied in a single housing or case 20. The illustrated case 20 includes a generally horizontal portion 22 (which may include an inclined or angled face or upper surface for enhanced visibility and ergonomics) and a generally vertical portion 24. The spinning membrane separator drive unit 14 and the centrifugal separator 16 are shown as being incorporated into the generally horizontal portion 22 of the case 20, while the controller 18 is shown as being incorporated into the generally vertical portion 24.

### A. Spinning Membrane Separator Drive Unit

The blood processing device 10 includes a spinner support or spinning membrane separator drive unit 14 for accommodating a generally cylindrical spinning membrane separator 26 of the fluid flow circuit 12 (Figs. 2 and 3). U.S. Patent No. 5,194,145 (which is hereby incorporated herein by reference) describes an exemplary spinning membrane separator drive unit that would be suitable for incorporation into the blood processing device 10, but it should be understood that the spinning membrane separator drive unit 14 may be differently configured without departing from the scope of the present disclosure.

The illustrated spinning membrane separator drive unit 14 has a base 28 configured to receive a lower portion of the spinning membrane separator 26 and an upper end cap 30 to receive an upper portion of the spinning membrane separator 26. Preferably, the upper end cap 30 is positioned directly above the base 28 to orient a spinning membrane separator 26 received by the spinning membrane separator drive unit 14 vertically and to define a vertical axis about which the spinning membrane separator 26 is spun. While it may be advantageous for the spinning membrane separator drive unit 14 to vertically orient a spinning membrane separator 26, it is also within the scope of the present disclosure for the spinning membrane separator 26 to be differently oriented when mounted to the blood processing device 10.

In one embodiment, one of the base 28 and upper end cap 30 of the spinning membrane separator drive unit 14 is movable with respect to the other, which may allow differently sized spinning membrane separators 26 to be received by the spinning membrane separator drive unit 14. For example, the upper end cap 30 may be translated vertically with respect to the base 28 and locked in a plurality of different positions, with each locking position corresponding to a differently sized spinning membrane separator 26.

At least one of the base 28 and the upper end cap 30 is configured to spin one or more components of the spinning membrane separator 26 about the axis defined by the spinning membrane separator drive unit 14. The mechanism by which the spinning membrane separator drive unit 14 spins one or more components of the spinning membrane separator 26 may vary without departing from the scope of the present disclosure. In one embodiment, a component of the spinning membrane separator 26 to be spun includes at least one element configured to be acted upon by a magnet (e.g., a metallic material), while the spinning membrane separator drive unit 14 includes a magnet (e.g., a series of magnetic coils or semicircular arcs). By modulating the magnetic field acting upon the aforementioned element of the spinning membrane separator 26, the component or components of the spinning membrane separator 26 may be made to spin in different directions and at varying speeds. In other embodiments, different mechanisms may be employed to spin the component or components of the spinning membrane separator 26.

Regardless of the mechanism by which the spinning membrane separator drive unit 14 spins the component or components of the spinning membrane separator 26, the component or components of the spinning membrane separator 26 is/are preferably spun at a speed that is sufficient to create Taylor vortices in a gap between the spinning component and a stationary component of the spinning membrane separator 26 (or a component that spins at a different speed). Fluid to be separated within the spinning membrane separator 26 flows through this gap, and filtration may be dramatically improved by the creation of Taylor vortices.

### B. Centrifugal Separator

As for the centrifugal separator 16, it includes a centrifuge compartment 32 that receives a centrifugal separation chamber 36 of the fluid flow circuit 12 (Figs. 2 and 3), as well as other components of the centrifugal separator 16. Further details as to an exemplary configuration of the centrifugal separator are set forth in PCT Patent Application Publication No. WO 2018/053217 A1.

Fluid (e.g., anticoagulated whole blood) is introduced into the centrifugal separation chamber 36 by an umbilicus, with the fluid being separated into a layer of less dense components (e.g., platelet-rich plasma, in the case of blood being separated) and a layer of more dense components (e.g., packed red blood cells) within the centrifugal separation chamber 36 as a result of centrifugal forces as it rotates. Components of an interface monitoring system may be positioned within the centrifuge compartment 32 to oversee separation of fluid within the centrifugal separation chamber 36. The interface monitoring system may include a light source 50 and a light detector 52, which is positioned and oriented to receive at least a portion of the light emitted by the light source 50.

The orientation of the various components of the interface monitoring system depends at least in part on the particular configuration of the centrifugal separation chamber 36. In general, though, the light source 50 emits a light beam (e.g., a laser light beam) through the separated fluid components within the centrifugal separation chamber 36 (which may be formed of a material that substantially transmits the light or at least a particular wavelength of the light without absorbing it). A portion of the light reaches the light detector 52, which transmits a signal to the controller 18 that is indicative of the location of an interface between the separated fluid components. If the controller 18 determines that the interface is in the wrong location (which can affect the separation efficiency of the centrifugal separator 16 and/or the quality of the separated fluid components), then it can issue commands to the appropriate components of the blood processing device 10 to modify their operation so as to move the interface to the proper location.

### C. Other Components Of The Blood Processing Device

In addition to the spinning membrane separator drive unit 14 and the centrifugal separator 16, the blood processing device 10 may include other components compactly arranged to aid fluid processing.

The generally horizontal portion 22 of the case 20 of the illustrated blood processing device 10 includes a cassette station 54, which accommodates a flow control cassette of the fluid flow circuit 12. In one embodiment, the cassette station 54 is similarly configured to the cassette station of U.S. Patent No. 5,868,696 (which is hereby incorporated herein by reference), but is adapted to include additional components and functionality. The illustrated cassette station 54 includes a plurality of clamps or valves V1-V9 (which are collectively referred to herein as the "valve system" of the blood processing system 10), which move between a plurality of positions (e.g., between a retracted or lowered position and an actuated or raised position) to selectively contact or otherwise interact with corresponding valve stations of the flow control cassette of the fluid flow circuit 12. Depending on the configuration of the fluid flow circuit 12, its cassette may not include a valve station for each valve V1-V9 of the cassette station 54, in which case fewer than all of the valves V1-V9 will be used in a fluid processing procedure.

In the actuated position, a valve V1-V9 engages the associated valve station to prevent fluid flow through that valve station (e.g., by closing one or more ports associated with the valve station, thereby preventing fluid flow through that port or ports). In the retracted position, a valve V1-V9 is disengaged from the associated valve station (or less forcefully contacts the associated valve station than when in the actuated position) to allow fluid flow through that valve station (e.g., by opening one or more ports associated with the valve station, thereby allowing fluid flow through that port or ports). Additional clamps or valves V10 and V11 of the valve system may be positioned outside of the cassette station 54 to interact with portions of valve stations (which may be lengths of tubing) of the fluid flow circuit 12 to selectively allow and prevent fluid flow therethrough. The valves V1-V9 and corresponding valve stations of the cassette station 54 and cassette may be differently configured and operate differently from the valves V10 and V11 and the valve stations that are spaced away from the cassette station 54.

The cassette station 54 may be provided with additional components, such as pressure sensors A1-A4, which interact with sensor stations of the cassette to monitor the pressure at various locations of the fluid flow circuit 12. For example, if the fluid source is a human donor, one or more of the pressure sensors A1-A4 may be configured to monitor the pressure of the donor's vein during blood draw and return. Other pressure sensors A1-A4 may monitor the pressure of the spinning membrane separator 26 and the centrifugal separation chamber 36. The controller 18 may receive signals from the pressure sensors A1-A4 that are indicative of the pressure within the fluid flow circuit 12 and, if a signal indicates a low- or highpressure condition, the controller 18 may initiate an alarm or error condition to alert an operator to the condition and/or to attempt to bring the pressure to an acceptable level without operator intervention.

The blood processing device 10 may also include a plurality of pumps P1-P6 (which are collectively referred to herein as the "pump system" of the blood processing device 10) to cause fluid to flow through the fluid flow circuit 12. The pumps P1-P6 may be differently or similarly configured and/or function similarly or differently from each other. In the illustrated embodiment, the pumps P1-P6 are configured as peristaltic pumps, which may be generally configured as described in U.S. Patent No. 5,868,696. Each pump P1-P6 engages a different tubing loop extending from a side surface of the flow control cassette and may be selectively operated under command of the controller 18 to cause fluid to flow through a portion of the fluid flow circuit 12. In one embodiment, all or a portion of the cassette station 54 may be capable of translational motion in and out of the case 20 to allow for automatic loading of the tubing loops into the associated pump P1-P6. In another exemplary embodiment, rather than employing peristaltic pumps, pneumatic pumps may be employed, with actuators incorporated into the cassette station 54 interacting with suitably configured portions of the fluid flow circuit 12 (e.g., pump stations of a cassette mounted to the cassette station 54) to convey fluid through the fluid flow circuit 12.

The illustrated blood processing device 10 also includes a centrifuge fluid sensor M1 for determining one or more properties of a fluid flowing into or out of a centrifugal separation chamber 36 mounted within the centrifugal separator 16. If the fluid flowing into the centrifuge separation chamber 36 is whole blood (which may include anticoagulated whole blood), the sensor M1 may be configured to determine the hematocrit of the blood flowing into the centrifuge separation chamber 36. If a fluid flowing out of the centrifuge separation chamber 36 is platelet-rich plasma, the sensor M1 may be configured to determine the platelet concentration of the platelet-rich plasma. The sensor M1 may detect the one or more properties of a fluid by optically monitoring the fluid as it flows through tubing of the fluid flow circuit 12 or by any other suitable approach. The controller 18 may receive signals from the sensor M1 that are indicative of the one or more properties of fluid flowing in fluid flow circuit 12 and use the signals to optimize the separation procedure based upon that property or properties. If the property or properties is/are outside of an acceptable range, then the controller 18 may initiate an alarm or error condition to alert an operator to the condition. A suitable device and method for monitoring hematocrit and/or platelet concentration is described in U.S. Patent No. 6,419,822 (which is incorporated herein by reference), but it should be understood that a different approach may also be employed for monitoring hematocrit and/or platelet concentration of fluid flowing through a fluid flow circuit.

The illustrated blood processing device 10 further includes a spinner outlet sensor M2, which accommodates tubing of the fluid flow circuit 12 that flows a separated fluid component out of the spinning membrane separator 26. The spinner outlet sensor M2 monitors the separated fluid component to determine one or more properties thereof, and may do so by optically monitoring the separated fluid component as it flows through the tubing or by any other suitable approach. In one embodiment, separated plasma flows through the tubing, in which case the spinner outlet sensor M2 may be configured to determine the amount of cellular blood components in the plasma and/or whether the plasma is hemolytic and/or lipemic. This may be done using an optical monitor of the type described in U.S. Patent No. 8,556,793 (which is hereby incorporated herein by reference) that measures the optical density of the fluid in the associated tubing, or by any other suitable device and/or method.

The illustrated blood processing device 10 also includes an air detector M3 (e.g., an ultrasonic bubble detector), which accommodates tubing of the fluid flow circuit 12 that flows fluid to a recipient. It may be advantageous to prevent air from reaching the recipient, whether a human recipient (e.g., the same human that serves as the blood source) or a non-human recipient (e.g., a storage bag or container), so the air detector M3 may transmit signals to the controller 18 that are indicative of the presence or absence of air in the tubing. If the signal is indicative of air being present in the tubing, the controller 18 may initiate an alarm or error condition to alert an operator to the condition and/or to take corrective action to prevent the air from reaching the recipient (e.g., by reversing the flow of fluid through the tubing or diverting flow to a vent location).

In one embodiment, the blood processing device 10 may include a platelet concentrate sensor M4 (Figs. 2 and 3). The platelet concentrate sensor M4 may be incorporated into the blood processing device 10 or may be an independent sensor. The platelet concentrate sensor M4 may be configured to determine the concentration of a concentrated platelet product exiting the spinning membrane separator 26. The sensor M4 may detect the concentration of the concentrated platelet product by optically monitoring the fluid as it flows through tubing of the fluid flow circuit 12 or by any other suitable approach.

The generally vertical portion 24 of the case 20 may include a plurality of volume measurement systems W1-W6 (six are shown, but more or fewer may be provided), each configured to be associated with one or more fluid containers F1-F7 of the fluid flow circuit 12 (Fig. 2). Each volume measurement system W1-W6 is configured to work in combination with the controller 18 to measure a current volume of fluid within an associated fluid container F1-F7 and to calculate a change in that volume between two or more points in time. The individual volume measurement systems W1-W6 may be variously configured without departing from the scope of the present disclosure, which may include two or more of the volume measurement systems W1-W6 being differently configured. In one exemplary embodiment, a volume measurement system W1-W6 may be configured as or include a weight scale configured to support and measure the weight of a fluid within an associated fluid container F1-F7 (with the measured weight being converted to a volume by a component of the volume measurement system W1-W6 or by the controller 18). In another exemplary embodiment, a volume measurement system W1-W6 may include one or more sensors configured to detect a volume and/or a change in volume of a fluid within an associated fluid container F1-F7. Volume measurement systems including additional components (e.g., both a weight scale and a sensor) and/or alternative components may also be employed without departing from the scope of the present disclosure.

Regardless of its particular configuration, each volume measurement system W1-W6 transmits to the controller 18 a signal that is indicative of the volume of the fluid within the associated container F1-F7 to track the change of volume during the course of a procedure. This allows the controller 18 to process the incremental volume changes to derive fluid processing volumes and flow rates and subsequently generate signals to control processing events based, at least in part, upon the derived processing volumes. For example, the controller 18 may diagnose leaks and obstructions in the fluid flow circuit 12 and alert an operator.

The illustrated case 20 is also provided with a plurality of hooks or supports H1 and H2 that may support various components of the fluid flow circuit 12 or other suitably sized and configured objects.

### D. Controller

According to an aspect of the present disclosure, the blood processing device 10 includes a controller 18, which is suitably configured and/or programmed to control operation of the blood processing device 10. In one embodiment, the controller 18 comprises a main processing unit (MPU), which can comprise, e.g., a Pentium^{™} type microprocessor made by Intel Corporation, although other types of conventional microprocessors can be used. In one embodiment, the controller 18 may be mounted inside the generally vertical portion 24 of the case 20, adjacent to or incorporated into an operator interface station (e.g., a touchscreen). In other embodiments, the controller 18 and operator interface station may be associated with the generally horizontal portion 22 or may be incorporated into a separate device that is connected (either physically, by a cable or the like, or wirelessly) to the blood processing device 10.

The controller 18 is configured and/or programmed to execute at least one biological fluid processing procedure but, more advantageously, is configured and/or programmed to execute a variety of different biological fluid processing procedures. For example, the controller 18 may be configured and/or programmed to carry out one or more of the following: a double unit red blood cell collection procedure, a plasma collection procedure, a plasma/red blood cell collection procedure, a red blood cell/platelet/plasma collection procedure, a platelet collection procedure, and a platelet/plasma collection procedure.

More particularly, in carrying out these fluid processing procedures, the controller 18 is configured and/or programmed to control one or more of the following tasks: drawing fluid into a fluid flow circuit 12 mounted to the blood processing device 10, conveying fluid through the fluid flow circuit 12 to a location for separation (i.e., into a spinning membrane separator 26 or centrifugal separation chamber 36 of the fluid flow circuit 12), separating the fluid into two or more components as desired, and conveying the separated components into storage containers, to a second location for further separation (e.g., into whichever of the spinning membrane separator 26 and centrifugal separation chamber 36 that was not used in the initial separation stage), or to a recipient (which may be a source from which the fluid was originally drawn).

This may include instructing the spinning membrane separator drive unit 14 and/or the centrifugal separator 16 to operate at a particular rotational speed and instructing a pump P1-P6 to convey fluid through a portion of the fluid flow circuit 12 at a particular flow rate. Hence, while it may be described herein that a particular component of the blood processing device 10 (e.g., the spinning membrane separator drive unit 14 or the centrifugal separator 16) performs a particular function, it should be understood that that component is being controlled by the controller 18 to perform that function.

Before, during, and after a procedure, the controller 18 may receive signals from various components of the blood processing device 10 (e.g., the pressure sensors A1-A4 and sensors M1-M4) to monitor various aspects of the operation of the blood processing device 10 and characteristics of the fluid and separated fluid components as they flow through the fluid flow circuit 12. If the operation of any of the components and/or one or more characteristics of the fluid or separated fluid components is outside of an acceptable range, then the controller 18 may initiate an alarm or error condition to alert the operator and/or take action to attempt to correct the condition. The appropriate corrective action will depend upon the particular error condition and may include action that is carried out with or without the involvement of an operator.

For example, the controller 18 may include an interface control module, which receives signals from the light detector 52 of the interface monitoring system. The signals that the controller 18 receives from the light detector 52 are indicative of the location of an interface between the separated fluid components within the centrifugal separation chamber 36. If the controller 18 determines that the interface is in the wrong location, then it can issue commands to the appropriate components of the blood processing device 10 to modify their operation so as to move the interface to the proper location. For example, the controller 18 may instruct one of the pumps P1-P6 to cause fluid to flow into the centrifugal separation chamber 36 at a different rate and/or for a separated fluid component to be removed from the centrifugal separation chamber 36 at a different rate and/or for the centrifugal separation chamber 36 to be spun at a different speed by the centrifugal separator 16.

If provided, an operator interface station associated with the controller 18 allows the operator to view on a screen or display (in alpha-numeric format and/or as graphical images) information regarding the operation of the system. The operator interface station also allows the operator to select applications to be executed by the controller 18, as well as to change certain functions and performance criteria of the system. If configured as a touchscreen, the screen of the operator interface station can receive input from an operator via touch-activation. Otherwise, if the screen is not a touchscreen, then the operator interface station may receive input from an operator via a separate input device, such as a computer mouse or keyboard. It is also within the scope of the present disclosure for the operator interface station to receive input from both a touchscreen and a separate input device, such as a keypad.

### II. The Disposable Fluid Flow Circuit

As for the fluid flow circuit or flow set 12 (Figs. 2 and 3), it is intended to be a sterile, single use, disposable item. Before beginning a given fluid processing procedure, the operator loads various components of the fluid flow circuit 12 in the case 20 in association with the blood processing device 10. The controller 18 implements the procedure based upon preset protocols, taking into account other input from the operator. Upon completing the procedure, the operator removes the fluid flow circuit 12 from association with the blood processing device 10. The portions of the fluid flow circuit 12 holding the collected fluid component or components (e.g., collection containers or bags) are removed from the case 20 and retained for storage, transfusion, or further processing. The remainder of the fluid flow circuit 12 is removed from the case 20 and discarded.

In the illustrated embodiment, the fluid flow circuit 12 includes a cassette, to which the other components of the fluid flow circuit 12 are connected by flexible tubing. The other components may include a plurality of fluid containers F1-F7. In the context of the present disclosure these containers include an anticoagulant container F1, a saline container F2, an in-process container F3, a return container F4, a plasma collection container F5, a platelet collection container F6, and an (optional) additive container F7. The illustrated flow circuit 12 further includes one or more fluid source access devices (e.g., a connector for accessing blood within a fluid container or a phlebotomy needle), a spinning membrane separator 26 and a centrifugal separation chamber 36.

The flow control cassette provides a centralized, programmable, integrated platform for all the pumping and many of the valving functions required for a given fluid processing procedure. In one embodiment, the cassette is similarly configured to the cassette of U.S. Patent No. 5,868,696, but is adapted to include additional components (e.g., more tubing loops) and functionality.

In use, the cassette is mounted to the cassette station 54 of the blood processing device 10 so as to align each sensor station with an associated pressure sensor A1-A4 of the cassette station 54 and its valve stations with an associated valve V1-V9. Each valve station may define one or more ports that allow fluid communication between the valve station and another interior cavity of the cassette (e.g., a flow path). As described above, each valve V1-V9 is movable under command of the controller 18 to move between a plurality of positions (e.g., between a retracted or lowered position and an actuated or raised position) to selectively contact the valve stations of the cassette. In the actuated position, a valve V1-V9 engages the associated valve station to close one or more of its ports to prevent fluid flow therethrough. In the retracted position, a valve V1-V9 is disengaged from the associated valve station (or less forcefully contacts the associated valve station than when in the actuated position) to open one or more ports associated with the valve station, thereby allowing fluid flow therethrough.

A plurality of tubing loops extend from the side surface of the cassette to interact with pumps P1-P6 of the blood processing device 10. The different pumps P1-P6 may interact with the tubing loops of the cassette to perform different tasks during a procedure, but in the context of the present disclosure, a different one of the pumps P1-P6 may be configured to serve as an anticoagulant pump P1, a source pump P2, a centrifuge pump P3, an outlet pump P4, a recirculation pump P5, and a plasma pump P6. If the pumps P1-P6 are differently configured (e.g., if they are configured as pneumatic pumps), then the cassette may be differently configured (e.g., with pump stations aligned with pneumatic pump actuators) to allow for the pumps P1-P6 to convey fluid through the cassette.

Additional tubing extends from the side surface of the cassette to connect to the other components of the fluid flow circuit 12, such as the various fluid containers F1-F7, the spinning membrane separator 26, and the centrifugal separation chamber 36. The tubing connected to the centrifugal separator chamber 36 (which includes one inlet tube and two outlet tubes) may be aggregated into an umbilicus.

Various additional components may be incorporated into the tubing leading out of the cassette or into one of the cavities of the cassette. For example, a manual clamp 56 may be associated with a line or lines leading to the fluid source, a return line filter 58 (e.g., a microaggregate filter) may be associated with a line leading to a fluid recipient, and/or an air trap 62 may be positioned on a line upstream of the centrifugal separation chamber 36.

### III. Exemplary Blood Processing Procedure

An exemplary blood processing procedure according to the present disclosure will now be described.

Prior to processing, an operator selects the desired protocol (e.g., using an operator interface station, if provided), which informs the controller 18 of the manner in which it is to control the other components of the blood processing device 10 during the procedure. This may include first selecting one of a plurality of possible procedures that the system is capable of executing and then, after selecting the nature of the procedure, selecting one or more parameters to be in effect during the procedure. For example, this may include selecting a platelet collection procedure from among a variety of blood separation procedures and then selecting a total volume of blood to be processed or a target yield of platelets to be collected during the procedure. If the fluid source is a living source (e.g., a donor or patient), the operator may proceed to enter various parameters, such as the sex/height/weight of the source. In one embodiment, the operator may also enter one or more characteristics of the fluid to be processed, such as a platelet pre-count. However, as will be explained in greater detail herein, rather than requiring a platelet pre-count, the controller 18 may be programmed to calculate the platelet concentration of the blood of the fluid source during a procedure based on input from one of more of the sensors M1-M4.

Once the controller 18 has received the necessary input, it may proceed to instruct the operator to mount the fluid flow circuit 12 to the blood processing device 10. If there are any fluid containers (e.g., a platelet additive solution container) that are not integrally formed with the fluid flow circuit 12, they may be connected to the fluid flow circuit 12 (e.g., by piercing a septum of a tube of the fluid flow circuit 12 or via a luer connector), with the fluid flow circuit 12 then being mounted to the blood processing device 10 (including the fluid containers F1-F7 being associated with the volume measurement systems W1-W6, as appropriate). In one exemplary embodiment, each volume measurement system W1-W6 includes a weight scale associated with a hook from which a fluid container may be hung. In another exemplary embodiment, at least one of the volume measurement systems W1-W6 includes a weight scale associated with a horizontal platform or surface, with a container being placed onto the platform or surface for support while the weight scale sends signals indicative of the weight of the container (and its contents) to be sent to the controller 18 throughout the course of a procedure. In other embodiments, a fluid container may be associated with a volume measurement system omitting a weight scale, but including other means for measuring the volume of fluid within the container (e.g., one or more sensors).

Once the fluid flow circuit 12 has been fully mounted to the blood processing device 10, the controller 18 may proceed with an integrity check of the fluid flow circuit 12 to ensure that the various components of the fluid flow circuit 12 are properly connected and functioning. Following a successful integrity check, the fluid source is connected to the fluid flow circuit 12 (e.g., by connecting to a container of previously collected fluid or by phlebotomizing a donor), and the fluid flow circuit 12 may be primed (e.g., using saline pumped from a saline container F2 by operation of one or more of the pumps P1-P6 of the blood processing device 10).

After the fluid flow circuit 12 has been primed, fluid processing may begin. In a draw phase of an exemplary platelet collection procedure (which is illustrated in Fig. 3), blood is drawn into the fluid flow circuit 12 from a blood source. If the blood source is a donor, then blood may be drawn into the fluid flow circuit 12 through a single needle that is connected to the cassette by line L1. Line L1 may include a manual clamp 56 that may initially be in a closed position to prevent fluid flow through line L1. When processing is to begin, an operator may move the manual clamp 56 from its closed position to an open position to allow fluid flow through line L1.

The blood is drawn into line L1 by the source pump P2 of the blood processing device 10. Anticoagulant from the anticoagulant container F1 may be drawn through line L2 under action of the anticoagulant pump P1 and added to the blood at a junction of lines L1 and L2.

In the illustrated embodiment, valve V10 is open to allow anticoagulated blood to flow through line L3 and a cassette sensor station associated with pressure sensor A1, while valve V11 is closed to prevent fluid flow through line L4. If the blood source is a living body (e.g., a donor), the pressure sensor A1 may communicate with the controller 18 to monitor the pressure within the vein of the blood source.

The cassette includes two valve stations downstream of the source pump P2, with valve V2 being closed to prevent flow through line L5 and valve V1 being open to allow flow through line L6. A portion of the blood is directed through line L7 and a cassette sensor station associated with pressure sensor A3 to the in-process container F3 and the remainder is directed through line L8 toward the centrifuge pump P3, which controls the amount of blood that is directed to the centrifugal separation chamber 36 instead of the in-process container F3. In particular, the flow rate of the source pump P2 is greater than the flow rate of the centrifuge pump P3, with the difference therebetween being equal to the flow rate of blood into the in-process container F3. The flow rates may be selected such that the in-process container F3 is partially or entirely filled with blood at the end of the draw phase.

The blood pumped through line L8 by the centrifuge pump P3 passes through line 9, an air trap 62, a cassette sensor station associated with pressure sensor A2 (which works in combination with the controller 18 of the blood processing device 10 to monitor the pressure in the centrifugal separation chamber 36), and the centrifuge fluid sensor M1 before reaching the centrifugal separation chamber 36 of the fluid flow circuit 12. The centrifuge fluid sensor M1 may operate in combination with the controller 18 to determine a characteristic of the blood flowing into the centrifugal separation chamber 36, such as the hematocrit of the blood.

The centrifugal separator 16 of the blood processing device 10 manipulates the centrifugal separation chamber 36 to separate the blood in the centrifugal separation chamber 36 into platelet-rich plasma and packed red blood cells. In one embodiment, the centrifugal separation chamber 36 is rotated nominally at 4,500 rpm, but the particular rotational speed may vary depending on the flow rates of fluids into and out of the centrifugal separation chamber 36.

The packed red blood cells exit the centrifugal separation chamber 36 via line L10 and flow through line L11 into the return container F4. The packed red blood cells travel through the centrifuge fluid sensor M1 after they exit the centrifugal separation chamber 36, with the centrifuge fluid sensor M1 operating in combination with the controller 8 to determine a characteristic of the red blood cells. In one embodiment, the centrifuge fluid sensor M1 operates in combination with the controller 18 to determine the hematocrit of the packed red blood cells exiting the centrifugal separation chamber 36, which may be used to calculate the hematocrit of the blood entering the centrifugal separation chamber 36 (as will be described in greater detail).

Platelet-rich plasma is drawn out of the centrifugal separation chamber 36 via line L12 by the combined operation of the recirculation and outlet pumps P5 and P4 of the blood processing device 10. The platelet-rich plasma travels through the centrifuge fluid sensor M1 and line L12 until it reaches a junction, which splits into lines L13 and L14. The centrifuge fluid sensor M1 may operate in combination with the controller 18 to determine a characteristic of the platelet-rich plasma, such as the concentration of platelets in the platelet-rich plasma, which may be used to calculate the concentration of platelets in the blood entering the centrifugal separation chamber 36 (as will be described in greater detail).

The recirculation pump P5 is associated with line L13 and redirects a portion of the platelet-rich plasma to a junction, where it mixes with blood in line L8 that is being conveyed into the centrifugal separation chamber 36 by the centrifuge pump P3. Recirculating a portion of the platelet-rich plasma into the centrifugal separation chamber 36 with inflowing blood decreases the hematocrit of the blood entering the centrifugal separation chamber 36, which may improve separation efficiency. By such an arrangement, the flow rate of the fluid entering the centrifugal separation chamber 36 is equal to the sum of the flow rates of the centrifuge pump P3 and the recirculation pump P5. As the platelet-rich plasma drawn out of the centrifugal separation chamber 36 into line L13 by the recirculation pump P5 is immediately added back into the centrifugal separation chamber 36, the bulk or net platelet-rich plasma flow rate out of the centrifugal separation chamber 36 is equal to the flow rate of the outlet pump P4.

Line L14 ends at a junction, where it joins with lines L15 and L16. Valve V6 is closed to prevent fluid flow through line L16, thereby directing the separated platelet-rich plasma to the spinning membrane separator 26 via line L15. Before reaching the spinning membrane separator 26, the portion of the platelet-rich plasma conveyed through line L15 passes through a cassette sensor station associated with pressure sensor A4, which may monitor the pressure of the spinning membrane separator 26.

While valve V6 is typically closed, it may be selectively opened to divert all or a portion of the platelet-rich plasma from line L14 into and through line L16 and to the return container F4, if necessary. An example would be at the start of a procedure when separation is initializing and platelets are not yet exiting the centrifugal separation chamber 36, in which case the fluid conveyed through line L14 by the outlet pump P4 could be diverted to the return container F4.

The spinning membrane separator drive unit 14 of the blood processing device 10 manipulates the spinning membrane separator 26 to separate the platelet-rich plasma into platelet-poor plasma ("plasma") and platelet concentrate ("platelets"). Plasma is pumped out of the spinning membrane separator 26 via line L17 by the plasma pump P6 of the blood processing device 10. Valves V5, V6, V8, and V9 are closed to direct the separated plasma along line L18, through valve V4, and into the return container F4 (with the separated red blood cells). On the way to the return container F4, the plasma passes through spinner outlet sensor M2, which may cooperate with the controller 18 to determine one or more characteristics of the plasma, such as the amount of cellular blood components in the plasma and/or whether the plasma is hemolytic and/or lipemic.

The platelet concentrate is conveyed out of the spinning membrane separator 26 via line L19. The platelet concentrate passes through the platelet concentrate sensor M4, which may work in combination with the controller 18 to determine one or more characteristics of the platelet concentrate, such as the concentration of platelets in the platelet concentrate, which may be used to calculate the concentration of platelets in the blood entering the centrifugal separation chamber 36 (as will be described in greater detail).

There is no pump associated with line L19, so instead the flow rate at which the platelets exit the spinning membrane separator 26 is equal to the difference between the flow rates of the outlet pump P4 and plasma pump P6. Valve V8 is closed to prevent fluid flow through the line L20, thereby directing the flow of platelets along line L19, through the platelet concentrate sensor M4 and valve V7, and into the platelet collection container F6. Valve V8 may be selectively opened to allow fluid flow through line L20 and to a junction, where it joins the plasma flowing through line L18 to the return container F4, if necessary.

Depending on the yield or volume of platelets to be collected, the above-described draw stage may be repeated, with draw stages being alternated with return stages in which blood from the in-process container F3 is separated in the centrifugal separation chamber 36 while previously collected blood components in the return container F4 are returned to the blood source. During such return stages, the separated red blood cells and platelet-rich plasma may be variously routed through the fluid flow circuit 12, typically with an additional volume of platelets being collected in the platelet collection container F6 after being separated from platelet-poor plasma in the spinning membrane separator 26 (as during the draw stage). A platelet additive solution from the additive container F7 may be added to the collected platelets in the platelet collection container F6 before ending the procedure.

### IV. Determination Of Platelet Concentration Of The Blood Of The Blood Source

In accordance with an aspect of the present disclosure, the controller 18 is configured to monitor and estimate the platelet concentration of a source's circulating blood (C_{source}) throughout a blood processing procedure, such as a platelet separation and collection procedure. The platelet concentration of the source's blood is needed for the blood processing system to calculate the number of platelets collected (platelet yield) and for system safeguards to ensure that the source's platelet count does not drop below acceptable limits (e.g., 150e3 platelets/µL). Additionally, by estimating the platelet concentration of a source's circulating blood during a blood processing procedure, a platelet pre-count measurement is not required.

During a blood processing procedure, once the source's blood is in the fluid flow circuit 12 (e.g., during a draw phase of the type shown in Fig. 3), the controller 18 may estimate the platelet concentration of a source's circulating blood (C_{source}) based on values obtained by the various sensors and scales and other inputs associated with the system and the equations described herein. This calculation may be completed once (e.g., during an early stage of a procedure) or may be repeated throughout the course of a procedure in order to monitor the platelet concentration of the source's circulating blood, which will decrease as platelets are collected during the procedure.

As noted above, the controller 18 may determine C_{source} based on values obtained by the controller 18 from the various sensors and scales that are coupled to the controller 18 and other inputs associated with the system, along with the following exemplary equations:
${C}_{wbcent} = {C}_{prp} * \left(1-{H}_{wbcent}\right)$ [Equation 1], in which C_{wbcent} is the concentration of platelets in anticoagulated (unseparated) blood from the blood source flowing through the fluid flow circuit 12 (e.g., the blood entering the centrifugal separation chamber 36 during the draw phase shown in Fig. 3), Cₚᵣₚ is the concentration of platelets in platelet-rich plasma separated from the blood of the source (e.g., as detected by the centrifuge fluid sensor M1 during the draw phase shown in Fig. 3), and H_{wbcent} is the hematocrit of anticoagulated (unseparated) blood from the blood source flowing through the fluid flow circuit 12 (e.g., the blood entering the centrifugal separation chamber 36 during the draw phase shown in Fig. 3);
${H}_{wbcent} = \left({H}_{rbc}*{Q}_{rbc}\right) / {Q}_{wbcent}$ [Equation 2], in which H_{rbc} is the hematocrit of red blood cells separated from the blood of the source (e.g., as detected by the centrifuge fluid sensor M1 during the draw phase shown in Fig. 3), Q_{rbc} is the flow rate of the red blood cells separated from the blood of the source (e.g., as the red blood cells are exiting the centrifugal separation chamber 36 during the draw phase shown in Fig. 3), and Q_{wbcent} is the flow rate of the anticoagulated blood of the blood source flowing through the fluid flow circuit 12 (e.g., flowing into the centrifugal separation chamber 36 during the draw phase shown in Fig. 3);
WB% = Part WB / (Part WB + Part AC) [Equation 3], in which WB% is the percentage of the anticoagulated blood of the source flowing through the fluid flow circuit 12 that is the blood of the source, Part WB is the number of parts of whole blood in the anticoagulated blood, and Part AC is the number of parts of anticoagulant in the anticoagulated blood (e.g., with Part WB being 10 and Part AC being 1 when the anticoagulant ratio is 10:1); and
${C}_{source} = {C}_{wbcent} / WB %$ [Equation 4].

As WB% will always be less than 1, it will be seen from Equation 4 that C_{source} will always be greater than C_{wbcent}. This can be understood to be correct on account of the collected whole blood being diluted with anticoagulant solution and, thus, having a lower platelet concentration than the blood when not diluted with anticoagulant.

As noted above, Cₚᵣₚ can be determined by the centrifuge fluid sensor M1 working in combination with the controller 18 using optical-based measurements of the platelet-rich plasma exiting the centrifugal separation chamber 36. In one embodiment, Cₚᵣₚ can be determined by using optical measurements recorded by the centrifuge fluid sensor M1 and a method for platelet concentration estimation, such as described in U.S. Patent Application Publication No. 2023/0243746, which is hereby incorporated herein by reference. Any other approach for determining Cₚᵣₚ may be employed without departing from the scope of the present disclosure.

As noted above, H_{rbc} can be estimated by an optical sensor, such as the centrifuge fluid sensor M1. Alternatively, H_{rbc} may be assumed to be a constant, empirically derived value for all procedures (e.g., 85%). Any other approach for determining H_{rbc} may also be employed without departing from the scope of the present disclosure.

When there is a pump that is directly responsible for the flow of separated red blood cells through a fluid flow circuit 12, Q_{rbc} can be determined based on the operational rate of that pump. Alternatively, when there is no pump that is directly responsible for the flow of separated red blood cells, Q_{rbc} may be calculated by subtracting the flow rate of platelet-rich plasma exiting a separator from the rate at which whole blood is entering the separator (Q_{wbcent}). Any other approach to determining Q_{rbc} (e.g., dividing a change in the weight of a red blood cell collection container over an elapsed time) may be employed without departing from the scope of the present disclosure.

To determine C_{source}, Equations 1, 2, and 3 can be substituted into Equation 4 to produce Equation 5, below:
${C}_{source} = {C}_{prp} * \left(1-\left(\left({H}_{rbc}*{Q}_{rbc}\right)/{Q}_{wbcent}\right)\right) / WB %$ [Equation 5].

Alternatively, rather than C_{source} being calculated based on the concentration of platelets in platelet-rich plasma flowing through the fluid flow circuit 12 (Cₚᵣₚ), C_{source} may instead be calculated based on the concentration of platelets in platelet concentrate flowing through the fluid flow circuit 12 (C_{pc}). In one embodiment, C_{pc} can be determined by using optical measurements recorded by a platelet concentrate sensor M4 (associated with line L19 in the fluid flow circuit of Figs. 2 and 3) and a method for platelet concentration estimation, such as described in U.S. Patent Application Publication No. 2023/0243746. Any other approach for determining C_{pc} may be employed without departing from the scope of the present disclosure.

The relationship between Cₚᵣₚ and C_{pc} is shown in Equation 6, below:
${C}_{prp} = \left({C}_{pc}*{Q}_{pc}\right) / {Q}_{prp}$ [Equation 6], in which Q_{pc} is the flow rate of the platelet concentrate separated from the blood of the source (e.g., as the platelet concentrate is exiting the spinning membrane separator 26 during the draw phase shown in Fig. 3) and Qₚᵣₚ is the flow rate of the platelet-rich plasma separated from the blood of the source (e.g., as the platelet-rich plasma is flowing into the spinning membrane separator 26 during the draw phase shown in Fig. 3). Q_{pc} and Qₚᵣₚ may be predetermined flow rates or may be determined by the controller 18 based on data from the weight scales and/or the optical system or may otherwise be determined by any suitable approach without departing from the scope of the present disclosure.

Equation 6 can be substituted into Equation 5, resulting in the following equation: ${C}_{source} = \left(\left({C}_{pc}*{Q}_{pc}\right)/{Q}_{prp}\right) * \left(1-\left(\left({H}_{rbc}*{Q}_{rbc}\right)/{Q}_{wbcent}\right)\right) / WB %$ [Equation 7].

Using Equation 5 or Equation 7, the controller 18 may estimate the concentration of platelets circulating in a source's blood (C_{source}) throughout a blood processing procedure. In one embodiment, the controller 18 may be pre-programmed to determine C_{source} using Cₚᵣₚ and not using C_{pc} (e.g., using Equation 5 or a comparable equation). In another embodiment, the controller 18 may be pre-programmed to determine C_{source} using C_{pc} and not using Cₚᵣₚ (e.g., using Equation 7 or a comparable equation). In yet another embodiment, the controller 18 may be pre-programmed to determine C_{source} using either Cₚᵣₚ (e.g., using Equation 5 or a comparable equation) or C_{pc} (e.g., using Equation 7 or a comparable equation). If the controller 18 is programmed to use Cₚᵣₚ to determine C_{source} and to use C_{pc} to determine C_{source}, it may enable an operator to select which approach to use. Alternatively, if the controller 18 is programmed to use Cₚᵣₚ to determine C_{source} and to use C_{pc} to determine C_{source}, it may calculate C_{source} using each approach and then determine C_{source} to be an average of the two calculated values.

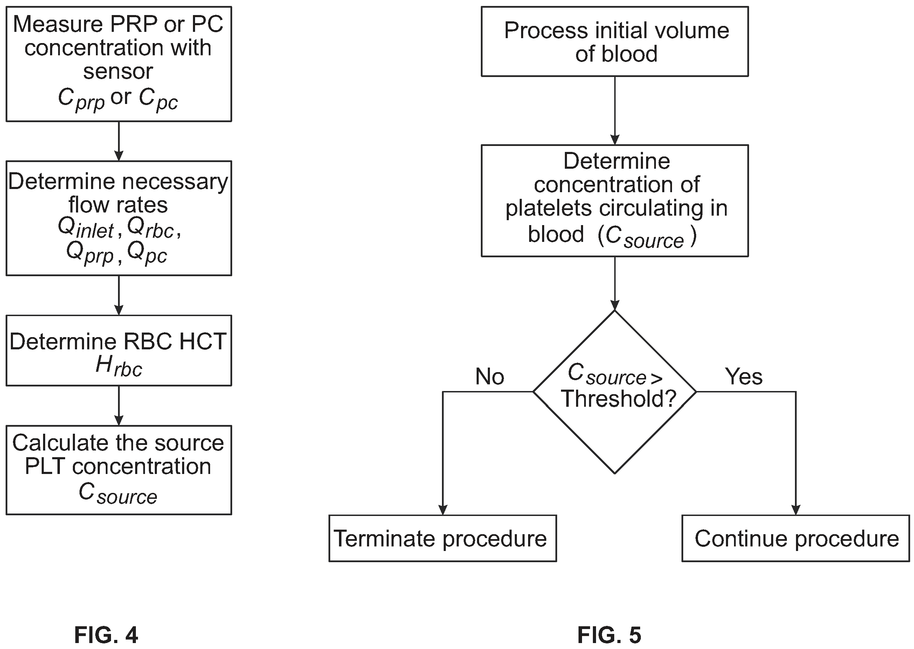

An exemplary implementation of the general approach to calculating C_{source} according to the present disclosure is illustrated in Fig. 4, though it should be understood that it may be implemented in other ways without departing from the scope of the present disclosure. For example, while Fig. 4 illustrates Cₚᵣₚ or C_{pc} being measured before other values are obtained, Cₚᵣₚ or C_{pc} may be measured after one or more of the other values are obtained. In another embodiment, all of the necessary values may be obtained substantially simultaneously by the controller 18, rather than being sequentially obtained. Additionally, as noted above, the controller 18 may repeatedly estimate the platelet concentration of a source's circulating blood throughout an entire procedure (in which case the method shown in Fig. 4 would be repeated) or may do so only once (e.g., during an early stage of the procedure).

### V. Determination Of Eligibility For Platelet Collection Procedure

The controller 18 may be programmed to compare the platelet concentration of a source's circulating blood to a predetermined limit or threshold. The predetermined limit or threshold may be pre-programmed into the controller 18 or the controller 18 may be provided with a minimum limit or threshold (e.g., by an operator or by a data management system) before initiating a procedure.

The controller 18 may be programmed such that, whenever the platelet concentration of the source's circulating blood is less than a predetermined limit or threshold (e.g., less than 100e3 platelets/µL or less than 150e3 platelets/µL), the controller 18 generates an alarm or otherwise alerts an operator, optionally giving the operator the option of terminating the procedure or continuing with a reduced target yield of platelets to be collected. In another embodiment, if C_{source} drops below a predetermined limit or threshold, the controller 18 may automatically end the procedure (which approach is shown in Fig. 5) or automatically reduce the target yield of platelets to be collected.

The minimum limit or threshold may be selected as a basis for determining whether the source is eligible for a procedure, with the source being deemed "eligible" when C_{source} exceeds the minimum limit or threshold and being deemed "ineligible" when C_{source} is less than the minimum limit or threshold. As noted above, C_{source} will decrease during the course of a procedure (as platelets continue to be separated from the blood of the source and collected), such that C_{source} may exceed the minimum limit or threshold during an early stage of a procedure (and, thus, the source will be deemed "eligible" to continue) and C_{source} may be less than the same minimum limit or threshold later in the procedure (and, thus, the source will be deemed "ineligible" to continue). In one embodiment, the controller 18 may be programmed to inform an operator that C_{source} is approaching the minimum limit or threshold. For example, if the predetermined limit or threshold is 100e3 platelets/µL, the controller may be programmed to alert a operator when C_{source} drops to 120e3 platelets/µL. In this instance, the operator can adjust the target final platelet yield for the procedure or terminate the procedure or allow the procedure to continue as originally intended (e.g., if the procedure is close to completion).

In another embodiment, the minimum limit or threshold may decrease one or more times during a procedure, as the remaining yield or volume of platelets to be collected decreases, which may allow a source to continue and complete a procedure that they would otherwise be deemed later in the procedure to be "ineligible" to complete. This may include, for example, the minimum limit or threshold decreasing continuously throughout a procedure or decreasing continually throughout a procedure. In a procedure employing multiple cycles in which platelets are collected, the minimum limit or threshold may decrease after each completed cycle (or, alternatively, after completion of only certain cycles), with the magnitude of the decrease being the same each time or different after completion of at least two of the cycles.

### VI. Aspects

Aspect 1. A blood processing device comprising: a pump system; a valve system; and a controller programmed to control the operation of the pump system and the valve system to execute a platelet collection procedure for a blood source, wherein the controller is programmed to determine a concentration of platelets circulating in blood of the blood source during the platelet collection procedure without using a platelet pre-count, compare the concentration of platelets circulating in the blood of the blood source during the platelet collection procedure to a minimum threshold, allow the platelet collection procedure to continue when the concentration of platelets circulating in the blood of the blood source exceeds the minimum threshold, and generate an alert or automatically terminate the platelet collection procedure when the concentration of platelets circulating in the blood of the blood source does not exceed the minimum threshold.

Aspect 2. The blood processing device of Aspect 1, wherein the controller is programmed to reduce the minimum threshold at least once during the platelet collection procedure.

Aspect 3. The blood processing device of any one of the preceding Aspects, wherein the controller is programmed to determine the concentration of platelets circulating in the blood of the blood source during a draw phase of the platelet collection procedure.

Aspect 4. The blood processing device of any one of the preceding Aspects, wherein the controller is programmed to repeatedly determine the concentration of platelets circulating in the blood of the blood source during the platelet collection procedure.

Aspect 5. The blood processing device of any one of the preceding Aspects, further comprising a sensor configured to optically detect a characteristic of the blood of the blood source or a separated component of the blood of the blood source during the platelet collection procedure, wherein the controller is programmed to use said characteristic to determine the concentration of platelets circulating in the blood of the blood source during the platelet collection procedure.

Aspect 6. The blood processing device of Aspect 5, wherein said characteristic comprises a concentration of platelets in platelet-rich plasma separated from the blood of the blood source during the platelet collection procedure.

Aspect 7. The blood processing device of Aspect 5, wherein said characteristic comprises a concentration of platelets in platelet concentrate separated from the blood of the blood source during the platelet collection procedure.

Aspect 8. The blood processing device of any one of Aspects 5-7, wherein said characteristic comprises a hematocrit of red blood cells separated from the blood of the blood source during the platelet collection procedure.

Aspect 9. The blood processing device of any one of Aspects 1-4, further comprising a first sensor configured to optically detect a hematocrit of red blood cells separated from the blood of the blood source during the platelet collection procedure, and a second sensor configured to optically detect a concentration of platelets in platelet-rich plasma separated from the blood of the blood source during the platelet collection procedure, wherein the controller is programmed to use the hematocrit of the red blood cells separated from the blood of the blood source during the platelet collection procedure and the concentration of platelets in the platelet-rich plasma separated from the blood of the blood source during the platelet collection procedure to determine the concentration of platelets circulating in the blood of the blood source during the platelet collection procedure.

Aspect 10. The blood processing device of any one of Aspects 1-4, further comprising a first sensor configured to optically detect a hematocrit of red blood cells separated from the blood of the blood source during the platelet collection procedure, and a second sensor configured to optically detect a concentration of platelets in platelet concentrate separated from the blood of the blood source during the platelet collection procedure, wherein the controller is programmed to use the hematocrit of the red blood cells separated from the blood of the blood source during the platelet collection procedure and the concentration of platelets in the platelet concentrate separated from the blood of the blood source during the platelet collection procedure to determine the concentration of platelets circulating in the blood of the blood source during the platelet collection procedure.

Aspect 11. A controller-implemented method of executing a platelet collection procedure for a blood source, the method comprising the controller: determining a concentration of platelets circulating in the blood of the blood source during the platelet collection procedure without using a platelet pre-count; comparing the concentration of platelets circulating in the blood of the blood source during the platelet collection procedure to a minimum threshold; allowing the platelet collection procedure to continue when the concentration of platelets circulating in the blood of the blood source exceeds the minimum threshold; and generating an alert or automatically terminating the platelet collection procedure when the concentration of platelets circulating in the blood of the blood source does not exceed the minimum threshold.

Aspect 12. The method of Aspect 11, wherein the controller is programmed to reduce the minimum threshold at least once during the platelet collection procedure.

Aspect 13. The method of any one of Aspects 11-12, wherein the controller determines the concentration of platelets circulating in the blood of the blood source during a draw phase of the platelet collection procedure.

Aspect 14. The method of any one of Aspects 11-13, wherein the controller repeatedly determines the concentration of platelets circulating in the blood of the blood source during the platelet collection procedure.

Aspect 15. The method of any one of Aspects 11-14, further comprising optically detecting a characteristic of the blood of the blood source or a separated component of the blood of the blood source during the platelet collection procedure, wherein the controller uses said characteristic to determine the concentration of platelets circulating in the blood of the blood source during the platelet collection procedure.

Aspect 16. The method of Aspect 15, wherein said characteristic comprises a concentration of platelets in platelet-rich plasma separated from the blood of the blood source during the platelet collection procedure.

Aspect 17. The method of Aspect 15, wherein said characteristic comprises a concentration of platelets in platelet concentrate separated from the blood of the blood source during the platelet collection procedure.

Aspect 18. The method of any one of Aspects 15-17, wherein said characteristic comprises a hematocrit of red blood cells separated from the blood of the blood source during the platelet collection procedure.

Aspect 19. The method of any one of Aspects 11-14, further comprising optically detecting a hematocrit of red blood cells separated from the blood of the blood source during the platelet collection procedure, and optically detecting a concentration of platelets in platelet-rich plasma separated from the blood of the blood source during the platelet collection procedure, wherein the controller uses the hematocrit of the red blood cells separated from the blood of the blood source during the platelet collection procedure and the concentration of platelets in the platelet-rich plasma separated from the blood of the blood source during the platelet collection procedure to determine the concentration of platelets circulating in the blood of the blood source during the platelet collection procedure.

Aspect 20. The method of any one of Aspects 11-14, further comprising optically detecting a hematocrit of red blood cells separated from the blood of the blood source during the platelet collection procedure, and optically detecting a concentration of platelets in platelet concentrate separated from the blood of the blood source during the platelet collection procedure, wherein the controller uses the hematocrit of the red blood cells separated from the blood of the blood source during the platelet collection procedure and the concentration of platelets in the platelet concentrate separated from the blood of the blood source during the platelet collection procedure to determine the concentration of platelets circulating in the blood of the blood source during the platelet collection procedure.

It will be understood that the embodiments described above are illustrative of some of the applications of the principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

## Claims

1. A blood processing device (10) comprising:
a pump system (P1-P6);
a valve system (V1-V11); and
a controller (18) programmed to control the operation of the pump system (P1-P6) and the valve system (V1-V11) to execute a platelet collection procedure for a blood source, wherein the controller (18) is programmed to
determine a concentration of platelets circulating in blood of the blood source during the platelet collection procedure without using a platelet pre-count,
compare the concentration of platelets circulating in the blood of the blood source during the platelet collection procedure to a minimum threshold,
allow the platelet collection procedure to continue when the concentration of platelets circulating in the blood of the blood source exceeds the minimum threshold, and
generate an alert or automatically terminate the platelet collection procedure when the concentration of platelets circulating in the blood of the blood source does not exceed the minimum threshold.

2. The blood processing device (10) of claim 1, wherein the controller (18) is programmed to reduce the minimum threshold at least once during the platelet collection procedure.

3. The blood processing device (10) of any one of the preceding claims, wherein the controller (18) is programmed to determine the concentration of platelets circulating in the blood of the blood source during a draw phase of the platelet collection procedure.

4. The blood processing device (10) of any one of the preceding claims, wherein the controller (18) is programmed to repeatedly determine the concentration of platelets circulating in the blood of the blood source during the platelet collection procedure.

5. The blood processing device (10) of any one of the preceding claims, further comprising a sensor (M1, M4) configured to optically detect a characteristic of the blood of the blood source or a separated component of the blood of the blood source during the platelet collection procedure, wherein the controller (18) is programmed to use said characteristic to determine the concentration of platelets circulating in the blood of the blood source during the platelet collection procedure.

6. The blood processing device (10) of claim 5, wherein said characteristic comprises a concentration of platelets in platelet-rich plasma separated from the blood of the blood source during the platelet collection procedure.

7. The blood processing device (10) of claim 5, wherein said characteristic comprises a concentration of platelets in platelet concentrate separated from the blood of the blood source during the platelet collection procedure.

8. The blood processing device (10) of any one of claims 5-7, wherein said characteristic comprises a hematocrit of red blood cells separated from the blood of the blood source during the platelet collection procedure.

9. The blood processing device (10) of any one of claims 1-4, further comprising
a first sensor (M1) configured to optically detect a hematocrit of red blood cells separated from the blood of the blood source during the platelet collection procedure, and
a second sensor (M4) configured to optically detect a concentration of platelets in platelet-rich plasma separated from the blood of the blood source during the platelet collection procedure, wherein the controller (18) is programmed to use the hematocrit of the red blood cells separated from the blood of the blood source during the platelet collection procedure and the concentration of platelets in the platelet-rich plasma separated from the blood of the blood source during the platelet collection procedure to determine the concentration of platelets circulating in the blood of the blood source during the platelet collection procedure.

10. The blood processing device (10) of any one of claims 1-4, further comprising
a first sensor (M1) configured to optically detect a hematocrit of red blood cells separated from the blood of the blood source during the platelet collection procedure, and
a second sensor (M4) configured to optically detect a concentration of platelets in platelet concentrate separated from the blood of the blood source during the platelet collection procedure, wherein the controller (18) is programmed to use the hematocrit of the red blood cells separated from the blood of the blood source during the platelet collection procedure and the concentration of platelets in the platelet concentrate separated from the blood of the blood source during the platelet collection procedure to determine the concentration of platelets circulating in the blood of the blood source during the platelet collection procedure.
